# EUROPÄISCHE PATENTANMELDUNG

(11) **EP 0 788 773 A1**
(43) Veröffentlichungstag der Anmeldung: **13.08.1997**
(21) Anmeldenummer: 97101786.8
(22) Anmeldetag: 05.02.1997
(51) Int. Cl.: A61B 17/17, A61B 17/16

(54) **Werkzeug zum Implantieren einer Endoprothese**

(30) Priorität: 08.02.1996 DE 19604494
(71) Anmelder: ZIMMER LIMITED, Swindon, Wiltshire SN2 6EA (GB)
(72) Erfinder: Zach, Wolfgang, 96486 Lautertal (DE)
(74) Vertreter: Eisenführ, Speiser & Partner

(57) **Zusammenfassung**

Es wird ein Werkzeug (2) angegeben, welches zum Implantieren einer Endoprothese in dem Hohlraum eines menschlichen Knochens dient. Das Werkzeug enthält mindestens einen Raspelkörper (30), welcher hinsichtlich seiner Form dem im Knochenhohlraum zu verankernden Prothesenschaft entspricht. Das Werkzeug ist dadurch gekennzeichnet, daß am proximalen Ende des Raspelkörpers ein Handgriff (4,6) lösbar befestigbar ist.

## Beschreibung

Die Erfindung betrifft ein Werkzeug zum Implantieren einer Endoprothese in den Hohlraum eines menschlichen Knochens, mit einem Raspelkörper, welcher eine Form aufweist, die dem im Knochenhohlraum zu verankernden Prothesenschaft entspricht.

Derartige Werkzeuge dienen dazu, dem Hohlraum, in welchem eine Endoprothese verankert werden soll, eine solche Form zu verleihen, daß der Prothesenschaft auf einfache Weise in den Hohlraum eingeführt und dort in geeigneter Weise zementfrei oder unter Zugabe von Knochenzement aufgenommen wird. Zu diesem Zweck wird der Hohlraum mit Raspelkörpern bearbeitet, welche in ihrer Form etwa der Form des zu verankernden Prothesenschaftes entsprechen. Um den notwendigen Knochenabtrag einerseits und die gewünschte Formanpassung andererseits in bequemer, zeitsparender Weise zu verwirklichen, wird ein entsprechend geformter Raspelkörper geeigneter Größe eingesetzt. In allen Fällen ist jedoch bei den bekannten Werkzeugen der Griff einstückig und fest am jeweiligen Raspelkörper angeordnet. Dadurch ist die Vorratshaltung der Werkzeuge wenig platzsparend und diese bekannten Werkzeuge lassen sich auch nur zum Raspeln einsetzen, sie müssen für nachfolgende Bearbeitungs- und Justiervorgänge wegen des dann störenden Handgriffs aus dem Knochenhohlraum entfernt werden.

Aufgabe der Erfindung ist es daher, ein Werkzeug der eingangs genannten Art derart weiterzubilden, daß das Werkzeug multifunktional für nachfolgende Bearbeitungsschritte und Justiervorgänge einsetzbar ist.

Diese Aufgabe wird bei dem Werkzeug der eingangs genannten Art erfindungsgemäß dadurch gelöst, daß am proximalen Ende des Raspelkörpers ein Handgriff lösbar befestigbar ist.

Die Vorteile der Erfindung liegen insbesondere darin, daß Handgriff und Raspelkörper zwei separate Teile darstellen, die - am proximalen Ende des Raspelkörpers - lösbar aneinander befestigt werden können. Dadurch lassen sich Raspelkörper mit unterschiedlichem Hieb an nur einen Griff ankoppeln; außerdem läßt sich zu jedem gewünschten Zeitpunkt der Handgriff abnehmen, während der Raspelkörper im Knochenhohlraum - in einer dem Prothesenschaft entsprechenden Position - steckt. Der Raspelkörper läßt sich dann als Gegenlager für einen nachfolgenden Bearbeitungsschritt, z.B. zum Planfräsen der Resektionskante des Knochens verwenden. Außerdem läßt sich alternativ der Raspelkörper auch als Testschaft benutzen, auf den der Endoprothese entsprechende Justierlehren oder Testadapter aufsetzbar sind, um die Position zu simulieren, welche die Endoprothese in dem Knochenhohlraum einnehmen würde. Die erfindungsgemäße Abnehmbarkeit des Griffes ermöglicht also, daß der Raspelkörper sowohl als Gegenlager und/oder Anschlag für weitere Bearbeitungsschritte sowie als Testschaft zur Überprüfung des korrekten Prothesensitzes dienen kann, sofern der Raspelkörper hinsichtlich seiner Form dem Schaft der zu implantierenden Endoprothese entspricht. Fehler bei der weiteren Bearbeitung sowie beim Überprüfen des Prothesensitzes können dadurch in einfacher Weise vermieden werden.

Besonders bevorzugt enthält das Werkzeug einen Testadapter, welcher einem am Prothesenschaft proximal anschließenden Teil der zu implantierenden Endoprothese entspricht, und der sich anstelle des Handgriffs auf das proximale Ende des Raspelkörpers aufsetzen läßt. Soll beispielsweise eine Hüftendoprothese implantiert werden, so besitzt der Testadapter die Form eines Prothesenkragens, gegebenenfalls des Prothesenhalses und des Prothesenkopfes. Der Testadapter kann einteilig oder mehrteilig ausgebildet sein, die Mehrteiligkeit kann beispielsweise so ausgebildet sein, daß ein separater Kragen, ein separater Hals und ein separater Kopf - beispielsweise entsprechend der Mehrteiligkeit der zu implantierenden Endoprothese - vorgesehen werden, so daß der Operateur die verschiedenen Parameter einer Prothese in situ simulieren und daraufhin die geeignete Prothese auswählen und implantieren kann. Falls es sich bei diesem Test zeigt, daß die gewünschte Position der Prothese noch nicht realisiert ist, läßt sich - außerordentlich einfach - der Testadapter abnehmen und der Griff wieder am Raspelkörper befestigen, anschließend läßt sich der Knochenhohlraum weiter bearbeiten, bis die gewünschte Position des Raspelkörpers und damit des Prothesenschaftes erreicht ist.

Vorteilhafterweise ist am proximalen Ende des Raspelkörpers ein Auflager vorgesehen, in welchem ein Stirnfräser lagerbar und betreibbar ist, nachdem der Handgriff vom Raspelkörper abgenommen wurde. Das Auflager für den Stirnfräser enthält bevorzugt eine unter vorgegebenem Winkel zur Längsachse des Raspelkörpers verlaufende ebene Stirnfläche, gegen welche der Stirnfräser aufliegt und mit den radial über dem Raspelkörper überstehenden Fräserschneiden die Stirnkante oder Resektionskante des den Raspelkörper umgebenden Knochens plan - mit der ebenen Stirnfläche fluchtend - abfräst. Bevorzugt enthält das Auflager senkrecht zur Stirnfläche darüber hinaus noch ein Sackloch oder einen Lagerzapfen, und an der Stirnfläche des Stirnfräsers ist ein entsprechender Lagerzapfen oder Sackloch angeordnet, um über eine Sackloch-/Zapfenverbindung eine radiale Führung zu verwirklichen, so daß der Stirnfräser sich im Zapfenlager geführt dreht.

Die Befestigung des Handgriffs am Raspelkörper läßt sich erfindungsgemäß mittels eines Bajonettverschlusses oder mittels einer Schraubverbindung verwirklichen. Zu diesem Zweck ist eine Koppelhülse vorgesehen, an die der Handgriff beispielsweise angeschweißt ist. Durch die Koppelhülse ist ein Stift zentral hindurchgeführt, der in dem Sackloch des Raspelkörpers verschraubt oder verspannt werden kann. Der Handgriff enthält einen Stiel, der sich bevorzugt parallel zur Längsachse des Raspelkörpers erstreckt und an seinem freien Ende in einem quer verlaufenden Griff endet.

Besonders bevorzugt lassen sich mehrere Raspelkörper mit unterschiedlichem Hieb und/oder gegebenenfalls unterschiedlicher Form an dem Handgriff ankoppeln.

Vorteilhafte Weiterbildungen der Erfindung sind durch die Merkmale der Unteransprüche gekennzeichnet.

Im folgenden werden Ausführungsbeispiele der Erfindung anhand der Zeichnung näher erläutert.

Es zeigen:
- Fig. 1: eine Seitenansicht eines Teils des erfindungsgemäßen Werkzeugs;
- Fig. 2: die Ankopplung des Griffs an dem Raspelkörper des Werkzeugs gemäß Fig. 1;
- Fig. 3: den Griff nach dem Ankoppeln am Raspelkörper;
- Fig. 4: eine Seitenansicht des Raspelkörpers und eines vom Raspelkörper abgenommenen Testadapters;
- Fig. 5: den Raspelkörper mit aufgesetztem Testadapter;
- Fig. 6: eine Seitenansicht des Raspelkörpers und eines mit dem Raspelkörper zusammenwirkenden Stirnfräsers;
- Fig. 7: den Raspelkörper mit auf dem Raspelkörper lagerndem Stirnfräser;
- Fig. 8: eine zweite Ausführungsform der Kopplung zwischen Raspelkörper und Griff in separiertem Zustand; und
- Fig. 9: eine der Fig. 8 entsprechende Darstellung des Raspelkörpers mit angekoppeltem Griff.

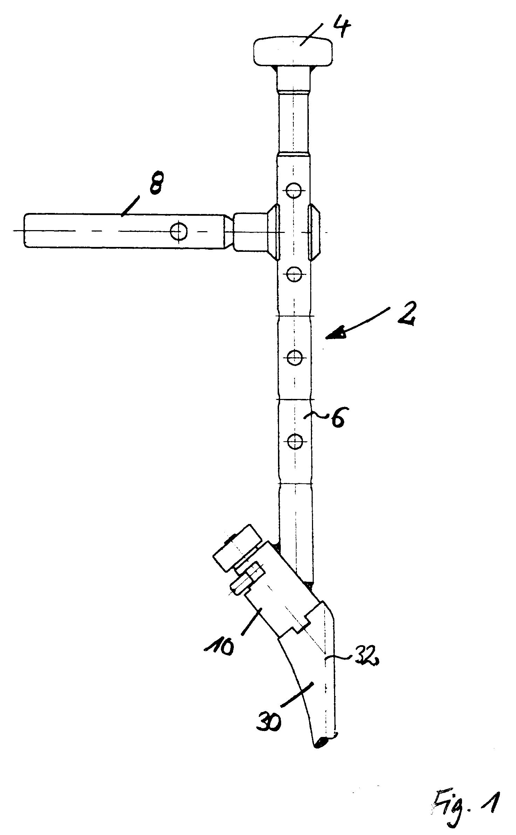

Die Fig. 1, 2 und 3 zeigen denjenigen Teil des Werkzeuges 2, der den Raspelkörper 30 und einen am Raspelkörper 30 lösbar befestigbaren Handgriff 4, 6 umfaßt. Der Raspelkörper 30 entspricht hinsichtlich seiner Form dem im Knochenhohlraum zu verankernden Prothesenschaft und verjüngt sich entsprechend zu seinem distalen Ende (nicht dargestellt) hin. Am proximalen Ende 39 des Raspelkörpers 30 ist eine Koppelhülse 10 lösbar befestigt, an der ein Stiel 6 befestigt ist, der an seinem oberen freien Ende einen Aufschlagteller 4 oder einen Anschluß eines motorischen Antriebs aufweist. Stiel 6 und Aufschlagteller 4 bilden den Handgriff, an dem zusätzlich ein weiterer Haltegriff 8 angesetzt werden kann. Wie in Fig. 1 dargestellt, verläuft die Längsachse des Stiels 6 mit geringem Parallelversatz fluchtend zur Längsachse 32 des Raspelkörpers, damit die vom Benutzer über den Handgriff auf den Raspelkörper 30 aufgebrachten Kräfte mit möglichst geringem Drehmoment wirken können.

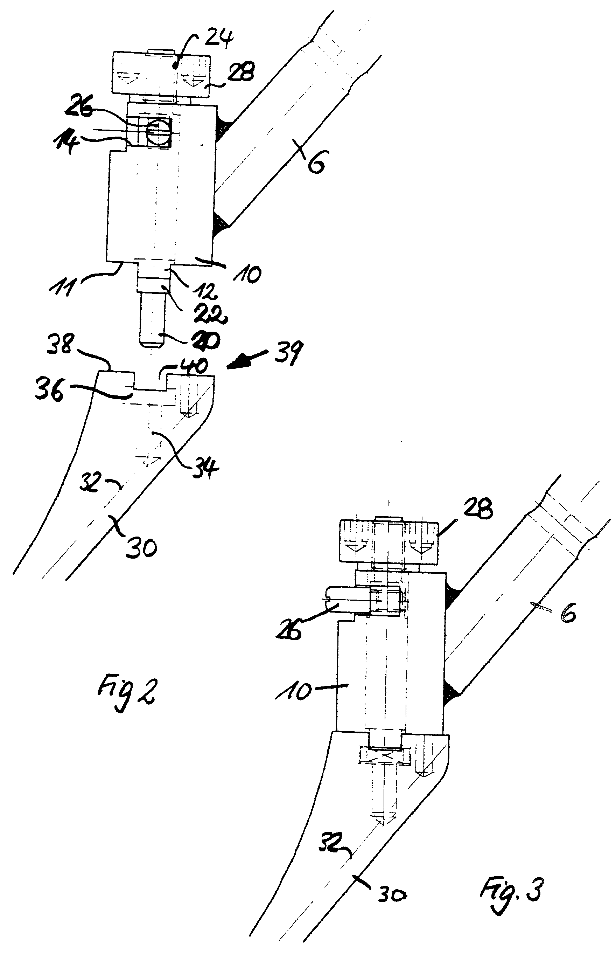

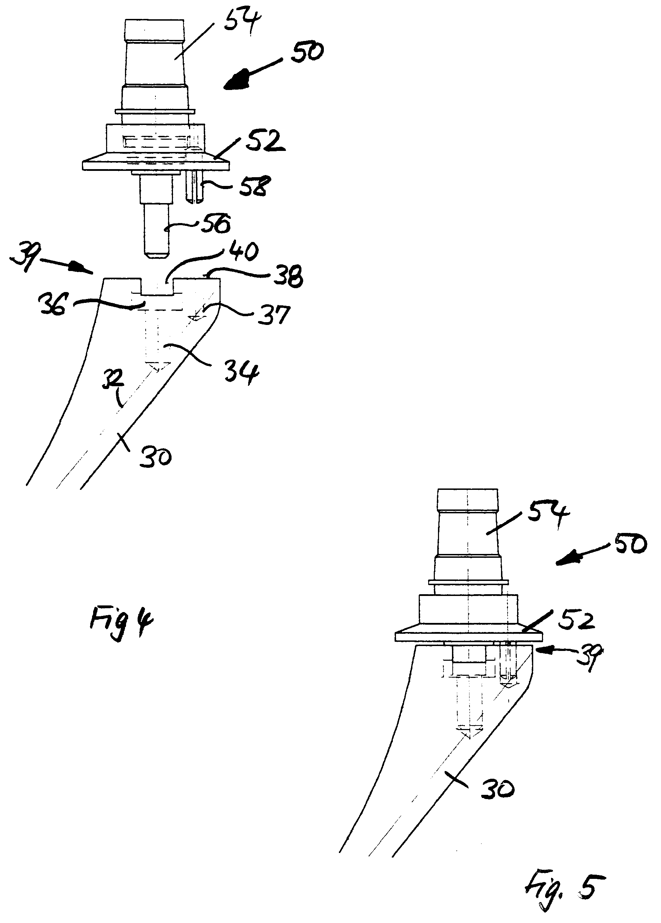

Wie insbesondere den Fig. 3 und 4 entnehmbar ist, besitzt der Raspelkörper 30 an seinem proximalen Ende eine ebene Stirnfläche 38, die unter einem vorgegebenen Winkel zur Längsachse 32 verläuft und eine Anlagefläche für an den Raspelkörper 30 ansetzbare oder ankoppelbare Teile des Werkzeugs besitzt. Die Stirnfläche 38 enthält in der in den Fig. 2 und 3 dargestellten Ausführungsform eine Quernut 40 zur Verdrehsicherung und zur Aufnahme der Schlagkräfte und ein von der Quernut 40 ausgehendes, zur Stirnfläche 38 senkrecht verlaufendes Sackloch 34, welches unmittelbar im Anschluß an die Quernut 40 eine Hinterschneidung 36 aufweist. Die an einem Ende des Handgriffs 4, 6 befestigte Koppelhülse 10 besitzt an ihrem unteren Ende eine ebene Auflagefläche 11 und einen Paßfederansatz 12, der in die Quernut 40 des Raspelkörpers eingreift, wenn die Anlagefläche 11 der Koppelhülse 10 sich gegen die Stirnfläche 38 des Raspelkörpers 30 anlegt. Durch die Koppelhülse 10 verläuft zentral ein Stift 20 mit einem unterhalb des Paßfederansatzes 12 liegenden Queransatz 22, der bei Drehung des zentralen Stiftes 20 sich in der Hinterschneidung 36 des Raspelkörpers 30 verankert und auf diese Weise die Koppelhülse mit dem Raspelkörper 30 verspannt. Zum Drehen des zentralen Stifts 20 ist ein Querzapfen 26 am Stift 20 befestigt, der durch eine entsprechende Nut 14 aus der Koppelhülse herausragt. Zum axialen Verspannen des Stifts 20 ist an dem Raspelkörper 30 abgewandten Ende des Stifts 20 eine Rändelschraube 28 vorgesehen, die auf einem Gewinde 24 des Stifts 20 läuft und den Stift in axialer Richtung gegen den Raspelkörper 30 verspannt, nachdem der Ansatz 22 die Hinterschneidung 36 hintergreift.

In den Fig. 4 und 5 ist der schon in den Fig. 2 und 3 gezeigte Raspelkörper 30 dargestellt, wobei jedoch der Handgriff 4, 6 samt Koppelhülse 10 abgenommen ist und anstelle des Handgriffs 4, 6 ein sogenannter Testadapter 50 auf das proximale Ende 39 des Raspelkörpers 30 aufsetzbar ist. Der Testadapter 50 entspricht in seiner Form einem an den Prothesenschaft proximal anschließenden Teil der zu implantierenden Endoprothese. Da in der dargestellten Ausführungsform der Raspelkörper 30 beispielsweise dem Schaft einer Hüftendoprothese entspricht, so entspricht der Testadapter dem Kragen 52 sowie dem Hals 54 einer entsprechenden Prothese. Auf den Hals 54 ist noch ein Kopf (nicht dargestellt) aufsetzbar, so daß die Position der zu implantierenden Prothese simuliert werden kann, wenn der Raspelkörper 30 im Knochenhohlraum verbleibt und als Testschaft dient.

Der Testadapter 50 besitzt in der dargestellten Ausführungsform einen Zapfen 56, der in das Sackloch 34 am proximalen Ende 39 des Raspelkörpers 30 einsetzbar ist. Vorgesehen ist außerdem ein Sicherungszapfen 58, der in eine Sicherungsbohrung 37 des Raspelkörpers 30 einsteckbar ist und ein Drehen des Testadapters 50 sowie eine Lockerung auf dem Raspelkörper verhindert.

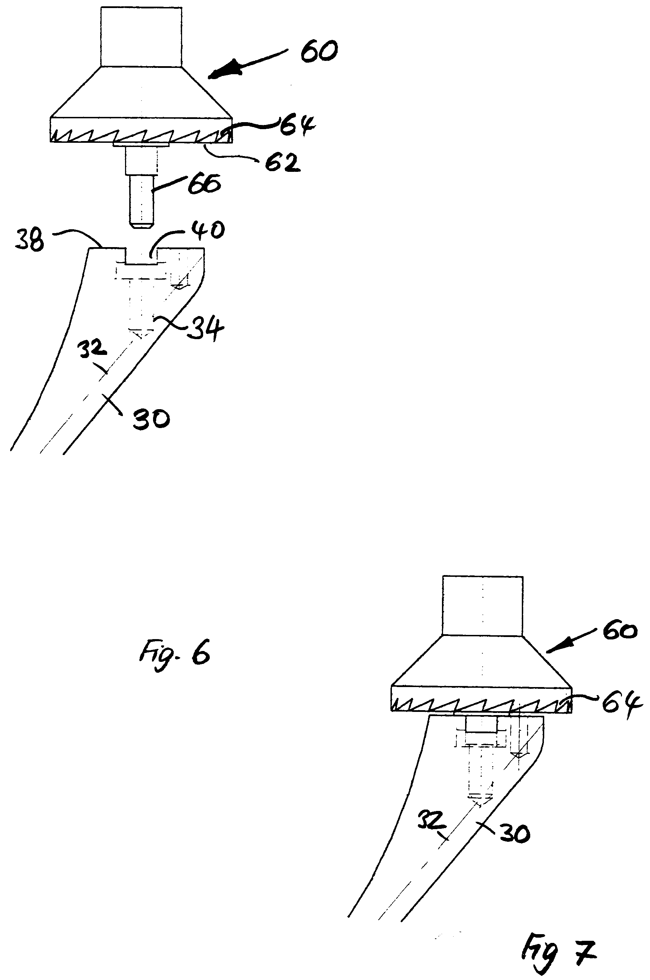

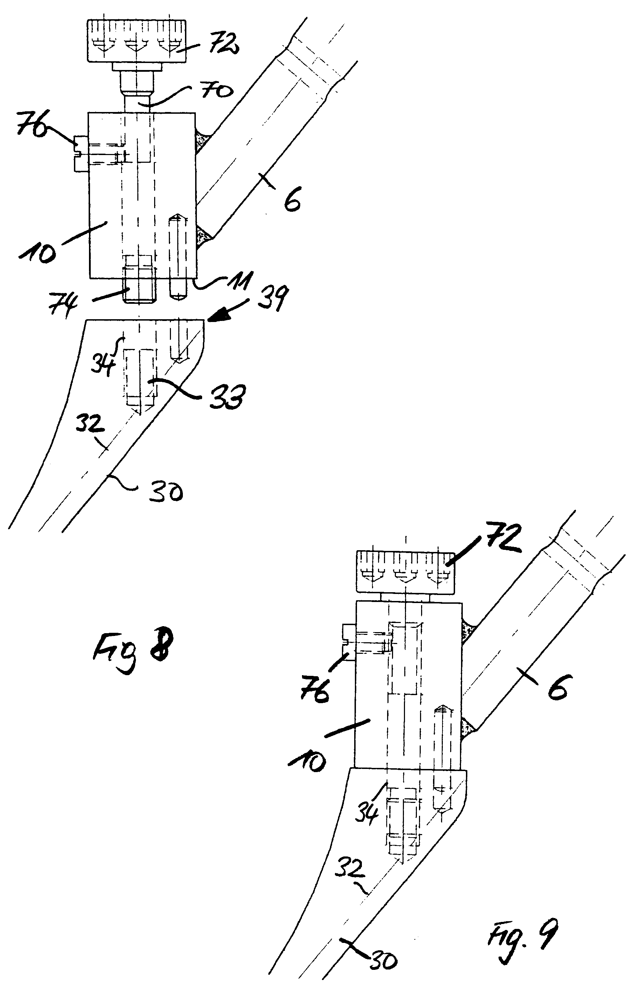

In den Fig. 6 und 7 ist der in den Fig. 1 bis 5 dargestellte Raspelkörper 30 gezeigt, der statt des Handgriffs 4, 6 bzw. statt des Testadapters 50 als Gegenlager für einen Stirnfräser 60 dient. Der Stirnfräser 60 besitzt an seiner unteren Stirnfläche 62 Schneiden 64, die radial über das proximale Ende des Raspelkörpers 30 hinausragen und zum Abfräsen des an den Raspelkörper anschließenden Knochenrandes dienen. Der Stirnfräser 60 besitzt an seiner Stirnfläche einen zentralen Zapfen 66, der mit ausreichemdem Spiel in das Sackloch 34 einführbar ist und dort drehbar lagert. Die Stirnfläche 38 und das Sackloch 34 bilden ein Auflager für den Stirnfräser 60, wenn der Raspelkörper 30 in dem Knochenhohlraum belassen ist, in den anschließend ein Prothesenschaft implantiert werden soll.

Die Fig. 8 und 9 zeigen eine den Fig. 2 und 3 entsprechende weitere Ausführungsform der Koppelverbindung zwischen Raspelkörper 30 und Griff 6. Das proximale Ende des Raspelkörpers 30 besitzt in dieser dargestellten Ausführungsform eine plane Stirnfläche 38, die unter einem vorgegebenen Winkel zur Längsachse 32 des Raspelkörpers 30 verläuft. Senkrecht zur Stirnfläche 38 ist ein Sackloch 34 eingearbeitet, welches in der dargestellten Ausführungsform ein Gewinde 33 aufweist. Die Koppelhülse 10 besitzt an ihrem unteren Ende eine ebene Anlagefläche sowie einen zentralen Stift 70, der an seinem unteren Ende ein mit dem Innengewinde 33 des Sackloches 34 kooperierendes Außengewinde 74 aufweist. Am gegenüberliegenden Ende besitzt der zentrale Stift 70 einen Kopf 72, an dem der zentrale Stift 70 in das Innengewinde 33 des Sackloches 34 eingeschraubt und auf diese Weise befestigt werden kann. Quer zum Stift 70 verläuft in der Hülse 10 eine Sicherungsschraube 76 als Verliersicherung des Stiftes 70.

## Patentansprüche

1. Werkzeug zum Implantieren einer Endoprothese in den Hohlraum eines menschlichen Knochens, mit einem Raspelkörper, welcher eine Form aufweist, die dem im Knochenhohlraum zu verankernden Prothesenschaft entspricht,
dadurch gekennzeichnet, daß am proximalen Ende des Raspelkörpers (30) ein Handgriff (4, 6) lösbar befestigbar ist.

2. Werkzeug nach Anspruch 1,
dadurch gekennzeichnet, daß ein Testadapter (50), welcher einem am Prothesenschaft proximal anschließenden Teil der zu implantierenden Endoprothese entspricht, anstelle des Handgriffs (4, 6) auf das proximale Ende (39) des Raspelkörpers (30) aufsetzbar ist.

3. Werkzeug nach Anspruch 2,
dadurch gekennzeichnet, daß der Testadapter (50) ein- oder mehrteilig ausgebildet ist.

4. Werkzeug nach einem der vorstehenden Ansprüche,
dadurch gekennzeichnet, daß der Raspelkörper (30) hinsichtlich seiner Form dem Schaft einer Hüftendoprothese entspricht, und daß der Testadapter (50) dem Kragen (52) und gegebenenfalls dem Hals (54) und gegebenenfalls dem Kopf der Hüftendoprothese entspricht.

5. Werkzeug nach einem der vorstehenden Ansprüche,
dadurch gekennzeichnet, daß anstelle des Handgriffs (4, 6) am proximalen Ende (30) des Raspelkörpers (30) ein den Raspelkörper (30) radial überragender Stirnfräser (60) aufsetzbar und drehbar lagerbar ist.

6. Werkzeug nach einem der vorstehenden Ansprüche,
dadurch gekennzeichnet, daß am proximalen Ende (39) des Raspelkörpers (30) ein Auflager (34, 38) für den Stirnfräser (60) vorgesehen ist.

7. Werkzeug nach Anspruch 6,
dadurch gekennzeichnet, daß das Auflager (34, 38) eine unter vorgegebenem Winkel zur Längsachse (32) des Raspelkörpers (30) verlaufende ebene Stirnfläche (38) enthält.

8. Werkzeug nach einem der vorstehenden Ansprüche:
dadurch gekennzeichnet, daß das Auflager (34, 38) senkrecht zur Stirnfläche (38) einen Lagerzapfen bzw. ein Sackloch (34) enthält, und daß an der Stirnfläche (62) des Stirnfräsers (60) ein entsprechendes Sackloch bzw. ein entsprechender Lagerzapfen (66) angeordnet ist.

9. Werkzeug nach einem der vorstehenden Ansprüche,
dadurch gekennzeichnet, daß die Stirnfläche (38) des Auflagers (34, 38) und die Stirnfläche (62) des Stirnfräsers (60) je ein Sackloch enthalten, in die ein separater Lagerzapfen einsetzbar ist.

10. Werkzeug nach einem der vorstehenden Ansprüche,
dadurch gekennzeichnet, daß die Befestigung des Handgriffs (4, 6) am Raspelkörper (30) mittels eines Bajonettverschlusses erfolgt.

11. Werkzeug nach einem der vorstehenden Ansprüche,
dadurch gekennzeichnet, daß die Befestigung des Handgriffs (4, 6) am Raspelkörper (30) mittels einer Schraubverbindung erfolgt.

12. Werkzeug nach einem der vorstehenden Ansprüche,
dadurch gekennzeichnet, daß eine Koppelhülse (10) vorgesehen ist, an der der Handgriff (4, 6) befestigbar ist, und daß durch die Koppelhülse (10) zentral ein Stift (20) hindurchgeführt ist, der in dem Sackloch (34) des Raspelkörpers (30) lösbar verschraubbar oder verriegelbar ist.

13. Werkzeug nach Anspruch 12,
dadurch gekennzeichnet, daß der Handgriff (4, 6) einen Stiel (6) enthält, der an der Koppelhülse (10) befestigt ist und sich parallel zur Längsachse (32) des Raspelkörpers (30) erstreckt.

14. Werkzeug nach Anspruch 1,
gekennzeichnet durch mehrere Raspelkörper (30) mit unterschiedlichem Hieb und/oder gegebenenfalls unterschiedlicher Form, die an dem Handgriff (4, 6) ankoppelbar sind.

15. Werkzeug nach Anspruch 14,
dadurch gekennzeichnet, daß mindestens einer der Raspelkörper (30) nach einem oder mehreren der Ansprüche 2 bis 13 ausgebildet ist.
